# EUROPEAN PATENT APPLICATION

(11) **EP 4 781 842 A1**
(43) Date of publication of application: **29.07.2026**
(21) Application number: 25154208.0
(22) Date of filing: 27.01.2025
(51) Int. Cl.: A24B 13/00, A24B 15/30, A61K 31/465

(54) **SMOKELESS ARTICLE**

(71) Applicant: Imperial Tobacco Limited, Bristol BS3 2LL (GB)
(72) Inventor: HOSSIENI, Hoda, Bristol, BS3 2LL (GB); NELSON, Luke, Bristol, BS3 2LL (GB); HALL, Steven Scott, Bristol, BS3 2LL (GB)
(74) Representative: Elkington and Fife LLP

(57) **Abstract**

A smokeless article (10) for oral delivery, the article comprising a pouch (12), wherein the pouch encloses a pouch content (14). A method of preparation of a smokeless article (10) and a kit comprising smokeless articles (10) is also described.

## Description

### FIELD

The present disclosure relates to a smokeless article and a method of manufacturing a smokeless article. In particular, the disclosure relates to a smokeless article for oral delivery comprising a pouch enclosing an oral nicotine delivery (OND) powder formulation, and method of producing thereof.

### BACKGROUND

Smokeless articles are placed in the mouth where saliva extracts the soluble element from the content contained within. Typically, the smokeless article is placed in the oral cavity, sublingually or in the oral vestibule (between the teeth and lips/cheeks). The user may assist extraction by oral manipulation, such as by chewing and/or sucking or pressing on the outside of the mouth to squeeze the pouch.

Some commercially available smokeless articles contain snuff. Snuff is smokeless tobacco made from ground or pulverised tobacco leaves. Snuff is available in dry form or wet (moist) form. Moist snuff may be referred to as snus. Two common varieties of snus are Scandinavian snus and American snus. Both varieties of snus are available in a loose form, but are often contained within a saliva permeable pouch.

There is a need for improved design of smokeless articles to enhance the user experience and improve the function of its constituent components.

It is against this background that the present invention has been developed.

### SUMMARY

At its most general, the present disclosure relates to a smokeless article e.g. an oral nicotine delivery (OND) article, for oral delivery. As used herein, the term "oral delivery" is intended to refer to any oral administration route achieved by placing the smokeless article into the oral cavity. This includes, but is not limited to, buccal, sub-lingual, periodontal, gingival and ingestion.

In a **first aspect,** the present disclosure provides a smokeless article for oral delivery comprising a pouch, wherein the pouch encloses a pouch content. The pouch content comprises dulcitol in an amount about 35% by weight of the total content or less. In some examples, the pouch content comprises nicotine in an amount from about 0.1 to about 5% by weight of the total content. In some examples, the pouch content comprises at least one filler material in an amount from about 1 to about 65% by weight of the total content. In some examples, the pouch content comprises water in an amount of about 1 to about 80% by weight of the total content.

Beneficially, when present in a pouch content, dulcitol helps to maintain the shape and integrity of the pouch and can also act as an additional bulking agent. This helps to ensure that the pouch does not break or leak during use, leading to a more consistent user experience. Additionally, the dulcitol in the pouch content may provide a matrix for nicotine delivery, allowing for the controlled release and hence efficient absorption of nicotine by the user, leading to an enhanced user experience.

Further advantageously, dulcitol serves as a sweetener, and so can enhance the overall taste and sweetness profile of the pouch content, leading to an enhanced user experience. Additionally, dulcitol is non-cariogenic and so does not contribute to tooth decay, and also has a low glycaemic index meaning that it has minimal impact on blood sugar levels. Therefore, the use of dulcitol in the pouch content can be seen to have several benefits compared with other sugars.

Additionally, the presence of dulcitol in a pouch content can result in a content with a whiter appearance compared with a content comprising e.g., xylitol. It is thought that this enhanced appearance of the content is as a result of the smaller crystal size of dulcitol compared with typical sugar alcohols (e.g., xylitol) used in pouch contents. A whiter pouch content leads to enhanced user perception of the content, and therefore to an improved user experience.

Dulcitol can also help to stabilise the various components of the pouch content, ensuring the even distribution of nicotine and any flavourants throughout the pouch, and can help maintain the moisture levels of the pouch. This may lead to a more consistent user experience and prolonged shelf life of the smokeless article.

Additionally, dulcitol provides a cooling sensation to the mouth when the smokeless article is in use. This enhances the overall sensory experience of the user.

The pouch content comprises dulcitol in an amount of about 35% by weight of the total content or less. In some examples, dulcitol is present in an amount of about 30% by weight of the total content or less, such as about 25% by weight of the total content or less, such as about 20% by weight of the total content or less. In some examples, dulcitol is present in an amount of about 0.1% by weight of the total content or more, such as about 1% by weight of the total content or more, such as about 5% by weight of the total content or more, such as about 10% by weight of the total content or more, such as about 15% by weight of the total content or more. In some examples, the amount of dulcitol present in the pouch content may be selected from a range with upper and lower amounts selected from the values given above. For example, the pouch content may comprise dulcitol in an amount from about 0.1 to about 35% by weight of the total content, such as from about 0.1 to about 30% by weight of the total content, such as from about 0.1 to about 25% by weight of the total content, such as from about 0.1 to about 10% by weight of the total content. In some examples, the pouch content comprises dulcitol in an amount from about 0.1 to about 10% by weight of the total content. In other examples, the pouch content may comprise dulcitol in an amount from about 0.1 to about 35% by weight of the total content, such as about 1 to about 35% by weight of the total content, such as from about 5 to about 35% by weight of the total content, such about from about 10 to about 35% by weight of the total content, such as from about 15 to about 35% by weight of the total content. In some examples, the pouch content comprises dulcitol in an amount of from about 15 to about 30% by weight of the total content.

By comprising dulcitol in an amount of about 35% by weight of the total content or less, such as from about 15 to about 30 % by weight of the total content, the dulcitol is able to enhance the sweetness profile of the pouch content without substantially altering the overall flavour of the content.

In some examples, the pouch content comprises an additional sugar alcohol. In some examples, the sugar alcohol is selected from the group xylitol, sorbitol, mannitol, erythritol, isomalt, glycerine, maltitol, lactitol, and inositol. In some examples, the pouch content comprises xylitol.

In some examples, the total amount of sugar alcohol (including dulcitol) present in the pouch content is from about 15 to about 30% by weight of the total content.

In some examples, the pouch content comprises a **nicotinic compound.** In some examples, the pouch content comprises a nicotinic compound in an amount from about 0.1% by weight of the total content or more, such as 1% by weight of the total content, such as 2% by weight of the total content or more. In some examples, a nicotinic compound is present in an amount of about 5% by weight of the total content or less, such as 4% by weight of the total content or less, such as 3% by weight of the total content or less. In some examples, the amount of nicotinic compound present in the pouch content may be selected from a range with upper and lower amounts selected from the values given above. For example, the pouch content may comprise a nicotinic compound in an amount from about 0.1 to about 4% by weight of the total content, such as 0.1 to about 3% by weight of the total content. In other examples, the pouch content may comprise a nicotinic compound in an amount from about 0.1 to about 5% by weight of the total content, such as about 1 to about 5% by weight of the total content, such as from about 2 to about 5% by weight of the total content. In some examples, the pouch content comprises a nicotinic compound in an amount from about 0.4 to about 2.4% by weight of the total content.

In some examples, the nicotinic compound may be selected from nicotine, nicotine salt(s), nicotine complex(es) and nicotine solvate(s). Alternatively or additionally, the nicotinic compound may be present within a plant material such as tobacco. In some examples, the nicotinic compound is nicotine. Such as freebase nicotine. In some examples, the nicotinic compound is nicotine polacrilex. In some examples, the pouch content comprises nicotine in an amount from about 0.1 to about 5% by weight of the total content, such as from about 0.4 to about 2.4% by weight of the total content.

In some examples, the pouch content comprises **filler material.** Filler materials may be provided to increase the volume of the smokeless article (e.g. by increasing the volume contained within the pouch and to strengthen the contents). In the present proposals, the nature of the filler materials may also impact the release profile of the nicotinic compound and the mouthfeel of the product. Filler materials may also be referred to as "fillers" or "bulking agents". For the avoidance of doubt, the filler material is intended to be distinct from the particles in the content as referred to herein.

In some examples, the filler material is at least one water-insoluble or water-soluble filler material. In some examples, the filler material is a water-insoluble filler material. In some examples, the filler material is a water-soluble filler material. In some examples, the filler material comprises a water-insoluble filler material and a water-soluble filler material. In some examples, the water-insoluble material has a water solubility of less than 0.1 g/100mL of water when measured at 25 °C and pH 7.0.

In some examples, the pouch content comprises filler material in an amount of from about 1% by weight of the total content, such as from about 10% by weight of the total content, such as from about 20% by weight of the total content, such as from about 30% by weight of the total content. In some examples, the pouch content comprises filler material in an amount of about 80% by weight of the total content or less, such as about 75% by weight of the total content or less, such as about 70% by weight of the total content or less. In some examples, the pouch content comprises filler material in an amount of about 65% by weight of the total content or less, such as about 60% by weight of the total content of less, such as about 50% by weight of the total content or less, such as about 40% by weight of the total content or less. In some examples, the pouch content comprises filler material in an amount selected from a range with upper and lower amounts selected from the values given above. For example, the pouch content may comprise filler material in an amount of from about 1 to about 80% by weight of the total content, such as from about 10 to about 80% by weight of the total content, such as from about 20 to about 80% by weight of the total content, such as from about 30 to about 80% by weight of the total content. In other examples, the pouch content may comprise filler material in an amount of from about 1 to about 75% by weight of the total content, such as from about 1 to about 60% by weight of the total content, such as from about 1 to about 50% by weight of the total content, such as from about 1 to about 40% by weight of the total content.

In some examples, the filler material is selected from the group comprising fibres, particles or spheres.

In some examples, the filler material comprises fibrous material. In some examples, the filler material comprises at least one fibrous material. In some examples the filler material comprises a combination of two or more types of fibrous material.

The fibrous material is composed essentially or completely of fibres. That is, in some examples, the filler material comprises fibres. The fibrous material may be insoluble in water, so that the fibrous material may comprise or consist of water-insoluble fibres. In some examples, water-insoluble fibres have a water solubility of less than 0.1 gram per 100 mL of water measured at 25 °C and at a pH of 7.0. The fibrous material may be food-grade. The fibrous material may be biocompatible.

The fibrous material may be naturally-occurring (e.g., a plant fibre), bio-derived, or synthetic. In some examples, the fibrous material is naturally-occurring or bio-derived, for example bio-derived. For example, a naturally-occurring fibrous material may be made of collagen, actin, fibrin, starch (e.g., corn starch), cellulose (e.g. powdered cellulose), alginate, or a polyhydroxyalkanoate (PHA), for example cellulose. For example, a bio-derived fibrous powder may be obtained by processing or modifying a natural product. For example, a bio-derived fibrous powder may be made of microcrystalline cellulose (MCC), poly(lactic acid) (PLA), poly(glycolic acid) (PGA), poly(ε-caprolactone), or polybutylene succinate (PBS), for example MCC. The fibrous material may consist of a combination of two or more fibrous materials.

In some examples, the fibrous material is cellulosic. Cellulosic fibres are fibres made of cellulose (e.g. powdered cellulose) or derived from cellulose (e.g. to form MCC).

In some examples, the fibrous material is colourless (e.g. it does not reflect light in the visible spectrum). In some examples, the fibrous material is white. In some examples, the fibrous material has been bleached. In some examples, the fibrous material has not been coloured by a colourant. In some examples, the fibrous material is a different colour to the particles.

In some examples, the fibrous material comprises or consists of fibres. In some examples, the fibrous material is not particulate. In some examples, the fibrous material comprises or consists of substantially non-spherical fibrous particles or fibres, a fibrous particle being a solid particle made from one or more fibres. In some examples, the fibrous material is powdered. In some examples, the fibrous material comprises or consists of elongate fibres or fibrous particles. In some examples, the fibrous material comprises or consists of fibrous particles, the fibrous particles being distinct from the particles referred to herein.

The fibrous material may comprise or consist of a plant fibre. A plant fibre is a naturally occurring fibrous material obtained from a plant. It may be chemically unmodified. The plant fibre may be a combination of fibres from different plants. In some examples, the plant fibre is essentially or completely water-insoluble. In some examples, the plant fibre comprises elongate fibres. The plant fibre may be included in the content to add strength and structure to the content of the pouch. Additionally, the plant fibre may function as a natural source of substances such as, for example, biologically/pharmacologically active compounds, flavourants, pH stabilisers etc. The plant fibre may, additionally or alternatively, modify the mouthfeel and/or release profile of the nicotinic compound.

For example, the plant fibre may comprise one or more selected form the group consisting of maize fibres, oat fibres, tomato fibres, barley fibres, rye fibres, sugar beet fibres, buck wheat fibres, wheat fibres, pea fibres, potato fibres, apple fibres, cocoa fibres, bamboo fibres, citrus fibres, pine fibres, eucalyptus fibres, and any combination thereof. In some examples, the plant fibre is selected from bamboo fibres, wheat fibres, or a combination thereof.

Additionally or alternatively, the plant fibre may comprise one or more selected from the group consisting of Amaranthus dubius, Arctostaphylos uva-ursi (Bearberry), Argemone mexicana, Amica, Artemisia vulgaris, Yellow Tees, Galea zacatechichi, Canavalia maritima (Baybean), Cecropia mexicana (Guamura), Cestrum noctumum, Cynoglossum virginianum (wild comfrey), Cytisus scoparius, Damiana, Entada rheedii, Eschscholzia califomica (California Poppy), Fittonia albivenis, Hippobroma longiflora, Humulus japonica (Japanese Hops), Humulus lupulus (Hops), Lactuca virosa (Lettuce Opium), Laggera alata, Leonotis leonurus, Leonurus cardiaca (Motherwort), Leonurus sibiricus (Honeyweed), Lobelia cardinalis, Lobelia inflata (Indian-tobacco), Lobelia siphilitica, Nepeta cataria (Catnip), Nicotiana species (Tobacco), Nymphaea alba (White Lily), Nymphaea caerulea (Blue Lily), Opium poppy, Passiflora incamata (Passionflower), Pedicularis densiflora (Indian Warrior), Pedicularis groenlandica (Elephant's Head), Salvia divinorum, Salvia dorrii (Tobacco Sage), Salvia species (Sage), Scutellaria galericulata, Scutellaria lateriflora, Scutellaria nana, Scutellaria species (Skullcap), Sida acuta (Wireweed), Sida rhombifolia, Silene capensis, Syzygium aromaticum (Clove), Tagetes lucida (Mexican Tarragon), Tarchonanthus camphoratus, Tumera diffusa (Damiana), Verbascum (Mullein), Zamia latifolia (Maconha Brava)

In some examples, the plant fibre comprises or consists of bamboo fibre. In some examples, the plant fibre comprises or consists of wheat fibre. In some examples, the fibrous material comprises MCC and one or both of bamboo fibre and wheat fibre.

As used herein, the term "bamboo fibre" refers to natural fibres from plants of the Bambusoideae subfamily of the Poaceae family of grasses.

As used herein, the term "wheat fibre" may refer to natural fibres from plants of the Triticum genus of the Poaceae family of grasses.

In some examples, the fibrous material consists of one or more non-tobacco plant fibres. In some examples, the fibrous material comprises a tobacco fibre and one or more other plant fibres.

In some examples, the fibrous material is a nicotine-dosed fibre. As used herein, the term "nicotine-dosed fibre" refers to a composition comprising a fibre and a nicotinic compound. The nicotinic compound may be added to or mixed with the fibre prior to incorporation into the smokeless article. In some examples, the fibres are loaded into a suitable dryer, sprayed with a solution of nicotinic compound and dried to form nicotine-dosed fibres. The dryer may be a fluidised bed dryer. The solution may be a solution of nicotinic compound in water or glycerin. In some examples, the solution of nicotinic compound may comprise from 10 to 50% nicotinic compound by weight of the total solution, for example from 10 to 40% or from 10 to 30% by weight of the total solution.

As used herein, the term "particle size" when referring to fibres indicates the average size of the longest dimension of the fibres, where the average is taken as the median (d50) in a distribution of the longest dimensions of a sample. For example, a particle size of 50 µm indicates that in the population of fibres, the median (d50) fibre length is 50 µm. Fibres of a desired particle size are available from commercial suppliers such as Jelu-werk. The particle size distribution can be determined using laser diffraction methods (such as described in ISO 13320:2020), by light/electron microscopy static image analysis (such as described in ISO 13322-1 :2014), or by sieving methods as described herein.

In some examples, the fibrous material has a monomodal particle size distribution.

The fibres of the fibrous material may have a particle size of 10 to 500 µm, for example 10 to 450 µm, 10 to 400 µm, 10 to 350 µm, 10 to 300 µm, 10 to 250 µm, 10 to 200 µm, 10 to 150 µm, 20 to 150 µm, 30 to 150 µm, 30 to 125 µm, 30 to 100 µm, 30 to 90 µm, 30 to 80 µm, 30 to 75 µm, 30 to 70 µm, 30 to 60 µm, 30 to 50 µm, 30 to 40 µm, 32 to 100 µm, 40 to 150 µm, 40 to 125 µm, 40 to 100 µm, 40 to 90 µm, 40 to 75 µm, 40 to 60 µm, 40 to 50 µm, 10 to 100 µm, 10 to 50 µm, 15 to 50 µm, 10 to 45 µm, 15 to 45 µm, 20 to 50 µm, 20 to 45 µm, 20 to 40 µm, or 25 to 35 µm. In some examples, the fibres have a particle size of about 30 µm. In some examples, the fibres have a particle size of about 40 µm. In some examples, the fibres have a particle size of about 50 µm.

In some examples, the fibres of the fibrous material have a particle size of 15 to 500 µm, for example, 20 to 500 µm, 50 to 500 µm, 50 to 400 µm, 50 to 350 µm, 100 to 350 µm, 150 to 350 µm, 200 to 350 µm, 200 to 300 µm, 225 to 300 µm, 225 to 275 µm, 240 µm to 260 µm, 245 µm to 255 µm, 150 to 250 µm, 160 to 240 µm, 170 to 230 µm, 180 to 220 µm, 190 to 210 µm, 195 to 205 µm, 100 to 500 µm, 150 to 500 µm, 200 to 500 µm, 250 to 500 µm, 250 to 450 µm, 250 to 400 µm, 250 to 350 µm, 260 to 340 µm, 270 to 330 µm, 280 to 320 µm, 290 to 310 µm, or 295 to 305 µm. In some examples, the fibres have a particle size of about 300 µm. In some examples, the fibres have a particle size of about 250 µm. In some examples, the fibres have a particle size of about 200 µm.

In some examples, the fibres of the fibrous material have a particle size of 25 µm or more, 30 µm or more, 32 µm or more, 35 µm or more, 40 µm or more, 45 µm or more, 50 µm or more, 60 µm or more, 70 µm or more, 75 µm or more, 80 µm or more, 90 µm or more, 100 µm or more, 150 µm or more, 175 µm or more, 200 µm or more, 225 µm or more, 250 µm or more, 275 µm or more, 300 µm or more, 325 µm or more, 350 µm or more, 400 µm or more, 500 µm or more, or 600 µm or more. In some examples, the fibres have a particle size of 600 µm or less, 500 µm or less, 450 µm or less, 400 µm or less, 375 µm or less, 350 µm or less, 325 um or less, 300 µm or less, 275 µm or less, 250 µm or less, 225 µm or less, 200 µm or less, 175 µm or less, 150 µm or less, 125 µm or less, 100 µm or less, 90 µm or less, 80 µm or less, 75 µm or less, 70 µm or less, 60 µm or less, 55 µm or less, 50 µm or less, 45 µm or less, 40 µm or less, or 35 µm or less.

In some examples, the fibres of the fibrous material have a particle size of 30 to 300 µm.

In some examples, the fibres of the fibrous material have a particle size of about 100 µm, 150 µm, 175 µm, 200 µm, 225 µm, 250 µm, 275 µm, 300 µm, 325 µm, 350 µm, 375 µm, or 400 µm. In some examples, the fibres have a particle size of about 200 µm. In some examples, the fibres have a particle size of about 250 µm. In some examples, the fibres have a particle size of about 300 µm.

The pouch content may comprise additional fillers. Suitable additional fillers include calcium carbonate, calcium phosphate, corn starch, grains, lactose, polysaccharides (e.g. maltodextrin), polyols, sugars (e.g. dextrose, mannitol, xylitol, sorbitol) and combinations thereof. In some cases, the additional filler content is 5 to 10% by weight of the total content e.g. around 6 to 9% by weight of the total content.

In some examples, the pouch content comprises tobacco. In some examples, the pouch comprises tobacco in an amount about 0.1% by weight of the total content. In some examples, the tobacco comprises reconstituted tobacco, cut tobacco or tobacco rag.

In some examples, the pouch content is substantially free from tobacco. By "substantially free from" it is meant that tobacco may be present in the pouch content in an amount of about 0.1% or less by weight of the total content, such as 0.01 wt.% or less by weight of the total content. In some examples, the content is free from tobacco. That is, in some examples, the content does not contain any tobacco. In this way, the user may experience a similar or enhanced recreational/pharmaceutical effect as compared to conventional tobacco-containing products without experiencing undesirable components inherent to tobacco (e.g. tobacco flavour).

In some examples, the pouch content comprises **particles.** That is, in some examples, the content comprises a plurality of particles. The particles referred to herein are distinct from the filler material contained in the content. For example, the particles described herein may form a particulate component of the content, which is distinct from, though may be intimately mixed with, other components (e.g. the at least one filler material) of the content. In some examples, the particles may also be referred to as grains, granules, beads, microbeads, etc. In some examples, the nature of the particles may have an effect on the blend/powder characteristics of the content, and/or on the user experience of the product. In some examples, the particles may be wax particles, i.e. particles made from a wax, or including a wax.

In some examples, the particles may be coloured particles, e.g. particles which have been coloured using one or more dyes or pigments. Such coloured particles may appear coloured to the human eye, reflecting a wavelength in the visible part of the electromagnetic spectrum. In some examples, the particles may be coloured wax particles, for example they may comprise one or more, typically one, selected from the group consisting of paraffin wax, Montan wax, carnauba wax, candelilla wax, beeswax, cottonseed wax, animal wax, microcrystalline wax, mineral wax, palm wax, rapeseed wax, and soy wax. In some examples, the particles are paraffin wax particles

In some examples, the reflected light has a wavelength of between 380-750 nm. In some examples, the reflected light has a colour selected from red, yellow, green and blue. In some examples, the colour of the coloured particles is selected from: red, orange, yellow, green, blue, indigo, violet, pink, brown, and grey. In some examples, all of the coloured particles have the same colour. In some examples, some of the particles have a different colour to other particles.

In some examples, a suitable colour for the coloured particles is selected based on the intended experience of the user, such as flavour enhancement. In some examples, the particles comprise a flavourant loaded thereon. In this way, the flavour can be associated with the particles and can alter the flavour release profile.

In some examples, the particles have an average particle size (d50) of between 200 and 2500 µm. In some examples, the particles have an average particle size (d50) of 400 µm or more. In some examples, the particles have an average particle size (d50) of 900 µm or less. In some examples, the particles have a melting point of 40 °C or greater. In some examples, the particles have a melting point of 100 °C or greater. In some examples, the particles have a density, as measured at 25 °C, of 0.4 g/cm³ or greater. In some examples, the particles have a density, as measured at 25 °C, of 0.9 g/cm³ or greater.

In some examples, the particles are contained in an amount by weight, based on the total weight of the content, of from about 0.1% to 10%. In some examples, the particles are contained in an amount of from about 1 % to about 3%. In this way, there may be a sufficient quantity of particles in the content to provide a content with beneficial characteristics. When coloured as described herein, the particles may provide a desirable speckled effect which may improve the user's experience. In some examples, the pouch content comprises a coloured particle in an amount of from about 0.1 to about 10% by weight of the total content.

In some examples, the pouch content may comprise one or more additional substance or component. Each additional substance/component may individually be a biologically/pharmacologically active compound, humectants, flavourants, fillers, preservatives, aqueous/non-aqueous solvents, and binders. Each additional substance/component may be provided for more than one purpose. That is, in some examples the pouch content further comprises at least one of a flavourant, humectant, sweetener, colourant, pH adjuster and solvent.

In some examples, the pouch content comprises a **flavourant.** Flavourants (also known as flavour components) may be provided in solid or liquid form, for example an extract, oil, butter, powder, gel, or concentrate. As used herein, the term "flavourant" denotes a compound having a desirable taste, aroma or both. Suitable flavourants include aloe vera extract, menthol, rosemary, chamomile, citrus, coconut, peppermint, frankincense, spearmint, wintergreen, tea tree oil, oregano, lavender eucalyptus, liquorice, chocolate, fruit flavour (including e.g. citrus, cherry etc.), vanilla, spice (e.g. ginger, cinnamon) and tobacco flavour. Other suitable flavourants include apple, apricot, banana, citrus peel oil, citrus peel juice, berries, guava, melon, papaya, peach, pear, peas, clove, ginger, mustard, spearmint oil, nuts, honey, soybean, coconut, passion fruit, olive, avocado, plum, rose apple, starfruit, sesame seed, tamarind, fig, cardamom, coriander seed and leaf, quince, lychee, dill, lovage, kiwi, loquat, sage, Chinese quince. The flavourant may be evenly dispersed throughout the content or may be provided in isolated locations and/or varying concentrations throughout the content. In some examples, the first flavourant may be loaded onto fibres and/or particles contained in the content. For example, the first flavourant may be loaded onto fibres and/or particles prior to forming the content. In some examples, the first flavourant may be mixed evenly within the content.

In some examples, the flavourant is bound to or loaded onto particles. In some examples, the flavourant is bound to or loaded onto the surface of the particles. In some examples, the flavourant is associated with the particles. In some examples, the flavourant is associated with pores of the particles. In some examples, the flavourant is loaded onto the particles before they are added to the content.

In some examples, the pouch content comprises a **biologically active or pharmacologically active compound.** Biologically/pharmacologically active compounds are provided to produce a pharmacological effect in the user. Suitable biologically/pharmacologically active compounds include the group consisting of: nicotine, caffeine, cathine and cathinone, kavalactones, mysticin, beta-carboline alkaloids, salvinorin A, together with any combinations, functional equivalents to, and/or synthetic alternatives of the foregoing. Biologically/pharmacologically active compounds may also have additive properties.

In some examples, the pouch content comprises a **solvent.** Solvents may be provided to improve the mouthfeel of the smokeless article and reduce the amount of saliva wetting required before the user experience begins. The addition of solvents to the content can also assist with the mixing of the components and ensure that a homogeneous coating of the bulking agent is achieved. Suitable solvents may be any solvent approved for use in food products in the UK and Europe, such as water, saline or water alcohol mixtures. In some examples, the solvent is water.

In some examples, the pouch content comprises a **salt.** Salts may be provided to maintain the osmolarity of the OND formulation within the smokeless article/pouch. Salts contribute to the overall positive user experience of the smokeless article, such as pleasant mouth feel. Examples of salts which may be used include sodium chloride, potassium chloride, magnesium chloride and calcium chloride.

In some examples, the pouch content comprises a **humectant.** Humectants may be provided to control moisture content thereby preventing the smokeless article from drying out during storage and reducing the amount of saliva wetting required before the user experience begins. Suitable humectants include polyhydric alcohols (e.g. propylene glycol (PG), triethylene glycol, 1,2-butane diol and glycerol such as vegetable glycerine (VG)) and their esters (e.g. glycerol mono-, di- or tri-acetate). The humectant component may consist of a single humectant compound or may consist of two or more different humectant compounds.

The humectant may have a lower limit of at least 1% based on the total weight of the content, such as at least 2%, such as at least 5%, such as at least 10%, such as at least 20%, such as at least 30%, or such as least 40%.

The humectant may have an upper limit of at most 70% based on the total weight of the content, such as at most 65%, such as at most 60%, or such as at most 20%, such as at most 10%, such as at most 5%, such as at most 2%.

In some examples, the amount of humectant is 1 to 70% based on the total weight of the content, such as 10 to 60%, such as 50 to 60%.

In some examples, the content has an amount of **water** greater than 5% by weight of the total content, such as 10% by weight of the total content or more, such as 20% by weight of the total content or more. In some examples, the content has an amount of water of 65% by weight of the total content or less, such as 60% by weight of the total content or less, such as 50% by weight of the total content or less, such as 40% by weight of the total content or less. For example, the content may have an amount of water of between 5 and 65% by weight of the total content, such as from 10 to 65% by weight of the total content, such as from 20 to 65% by weight of the total content. In some examples, the content has an overall amount of water of between 5 and 60% based on the total weight of the content, such as from 30 to 60%, such as from 35 to 60%, such as from 40 to 60%, such as from 50 to 60%. In some examples, the content has an overall amount of water from 40 to 55%, such as from 40 to 50% or from 42 to 50%, such as from 42 to 28%, based on the total weight of the content. In other examples, the content has an overall amount of water from 52 to 60% based on the total weight of the content.

Smokeless articles having a total moisture content of 10% or less are generally considered to be 'dry'. Smokeless articles having a total moisture content of 40% or more are generally considered to be `wet'.

The humectant in the pouch content (e.g. OND formulation) may comprise or consist of water and one or more polyhydric alcohols, for example water and glycerol. In some examples, the humectant may comprise propylene glycol.

In some examples, the pouch content comprises a **sweetener** in addition to the dulcitol present in the content. Sweeteners may be provided to modify the user taste perception and, in particular, overcome bitter flavours that result from other substances. Suitable sweeteners include honey, sugar, brown sugar, glucose, fructose, sucrose, aspartame, xylitol, maltitol, saccharin sodium, glycyrrhizin tripotassium liquorice, jujube, or a mixture thereof.

In some examples, the pouch content comprises a sweetener (e.g. acesulfame K) in an amount of from 0 to 1% based on the total weight of the content, for example from 0.01 to 1 %, from 0.01 to 0.5%, from 0.01 to 0.2%, from 0.01 to 0.1%, from 0.05 to 0.2%, or from 0.05 to 0.15% based on the weight of the total content. Such amounts being in addition to the dulcitol present in the content.

The amount of sweetener may have a lower limit of at least 0.01% by weight of the content such as at least 0.02%, such as at least 0.03%, such as at least 0.04%, such as at least 0.05%, or such as least 0.06%. Such amounts being in addition to the dulcitol present in the content.

In some examples, the pouch content comprises a **stabiliser.** Stabilisers are provided to prevent decomposition or degradation overtime during storage by, for example, retarding oxidation or unwanted biological activity. Stabilisers may be selected from the group consisting of antioxidants including vitamin E, such as tocopherole, ascorbic acid, sodium pyrosulfite, butylhydroxytoluene, butylated hydroxyanisole, edetic acid and salts thereof; and preservatives including citric acid, tartaric acid, lactic acid, malic acid, acetic acid, benzoic acid, sorbic acid and salts thereof.

In some examples, the pouch content comprises a **pH stabiliser or adjuster.** pH stabilisers or adjusters (pH modifiers) may be provided to adjust the user experience and/or modify the bioavailability of a pharmacologically active compound. For instance, under acidic conditions, nicotine is protonated and does not readily cross mucous membranes. Examples of suitable pH stabilisers include ammonia, ammonium carbonate, sodium carbonate and calcium carbonate. These components may have a buffering action, which helps to maintain the pH at the desired level. In some examples, the overall pH of the smokeless article is pH 7 to pH 9, such as pH 7.25 to pH 8.75 or pH 7.5 to pH 8.5, for example around pH 8.

The pH may be stable for at least 4 months, at least 5 months, at least 6 months, at least 7 months, or at least 8 months. The content (e.g., OND formulation) may have a shelf-life of at least 4 months, at least 5 months, at least 6 months, at least 7 months, or at least 8 months.

The overall pH of a smokeless article may be determined by, for example, (i) placing the smokeless article in 10 mL of distilled water (ii) agitating the mixture for at least 5 minutes and (iii) measuring the pH of the solution with a pH probe.

In some examples, the pouch content comprises a pH modifier (e.g. sodium carbonate) in an amount of from 0 to 1% based on the weight of the total content, for example from 0.01 to 1%, from 0.01 to 0.5%, from 0.01 to 0.2%, from 0.01 to 0.1%, from 0.01 to 0.08%, or from 0.01 to 0.06 % based on the weight of the total content.

The pH modifier may have a lower limit of at least 0.01 % based on the weight of the total content such as at least 0.02%, such as at least 0.03%, such as at least 0.04%, such as at least 0.05%, or such as least 0.06%.

In some examples, the pouch content comprises a **binder.** Suitable binders include starches and/or cellulosic binders such as methyl cellulose, ethyl cellulose, hydroxypropyl cellulose, hydroxyethyl cellulose and carboxymethyl cellulose, gums such as xanthan, guar, arabic and/or locust bean gum, organic acids and their salts such as alginic acid (sodium alginate), agar and pectins. In some examples the binder content is 5 to 10% based on the weight of the total content, such as around 6 to 9%, such as 7 to 8%.

In some examples, the pouch content comprises a colourant. Colourants may be provided to modify the user impression of the smokeless article. Colourants may include whitening agents. Colourants may be selected from one or more of common colourants such as curcumin (E100), turmeric (E100(ii)), riboflavin (E101), riboflavin-5'-phosphate (E101(ii)), tartrazine (E102), quinoline yellow (E104), riboflavin-5-sodium phosphate (E106), yellow 2G (E107), sunset yellow FCF (E110), carmine, cochineal (E120), azorubine (E122), amaranth (E123), ponceau 4R (E124), erythrosine (E127), red 2G (E128), allura red AC (E129), patent blue V (E131), indigotine (E132), brilliant blue FCF (E133) chlorophylls (E140), copper complexes of chlorophyll (E141), green S (E142), caramel (E150a-d), brilliant black BN (E151), carbon (E153), brown FK (E154), brown HT (E155), alfa-, beta- and gamma- carotene (E160a), annatto, bixin, norbixin (E160b), bell pepper (Paprika) extract (E160c), lycopene (E160d), beta- apo-8'-carotenal (E160e), ethyl ester of beta-apo-8'-carotenic acid (E160f), flavoxanthin (E161a), lutein (E161b), cryptoxanthin (E161c), rubixanthin (E161d), violaxanthin (E161e), rhodoxanthin (E161f), canthaxanthin (E1619), citranaxanthin (E161h), beetroot extract (E162), anthocyanins (E163), calcium carbonate (E170), titanium dioxide (E171), iron oxides (E172), aluminium (E173), silver (E174), gold (E175), lithol rubine BK (E180), tannins (E181). The amount of colourant may be, separately to any colourant associated with the particles described above, up to about 3% based on the weight of the total content, such as about 0.5% to about 2.5% or about 1% to about 2%.

In some examples, the pouch content has a bulk density of about 0.8 g/cm³ or less, such as 0.75 g/cm³ or less, such as 0.5 g/cm³ or less. In some examples, the pouch content has a bulk density of about 0.3 g/cm³ or more, such as about 0.4 g/cm³ or more. In some examples, the bulk density of the pouch content may be selected from a range with upper and lower values selected from the values given above. For example, the bulk density of the pouch may be from 0.3 to 0.8 g/cm³, such as 0.3 to 0.75 g/cm³ such as 0.4 to 0.5 g/cm³. The bulk density of the pouch content may be obtained by measuring the untapped mass and volume of the content.

The smokeless article comprises a pouch enclosing a pouch content, wherein the content is completely enclosed by the pouch. The pouch is sealed to ensure that the content of the pouch does not scatter inside the mouth.

The smokeless article may have a mass of about 0.1 g to 5.0 g, such as about 0.5 g to about 4.0 g or about 1.0 g to about 3.0 g.

The smokeless article may have a length of about 30 mm, such as about 28 mm or 26 mm, a width of about 12 mm, such as about 10 mm or 8 mm, and a depth of about 5 mm, such as about 4 mm or 3 mm.

The smokeless article may have an active lifetime of about 20 minutes to about 60 minutes, such as about 25 minutes to 50 minutes or about 30 minutes to about 45 minutes, after being placed in the mouth. As used herein, the term "active lifetime" is intended to refer to the amount of time after being placed in the mouth that the smokeless article provides the user with a perceptible taste and/or physiological experience. For example, for an article containing an active ingredient such as nicotine or other pharmacologically active ingredient the active lifetime may be defined as the in use period of time in which 90% of the available pharmacologically active is released. In other words, the active lifetime may be the duration of time from insertion into the oral cavity for 90% of the total amount of nicotine pharmacologically active ingredient that is capable of being released during normal use to dissolve into the user's saliva and /or enter the user's bloodstream. It will therefore be appreciated that the active lifetime of a product may vary from user to user and for a user based on oral conditions, in particular extent of salivation. Nonetheless, the skilled person is able to mimic oral conditions to determine the active lifetime in one instance, which can be used as a comparison or analysis point.

In some examples, the pouch may be formed from one or more materials. The pouch material may be formed from fiber, paper, cloth and fabric. The pouch material may be formed from one or more polymeric materials. The polymeric material may be selected from one or more of hydroxypropyl cellulose (HPC), hydroxypropyl methylcellulose (HPMC), polyvinyl alcohol (PVOH), polyvinylpyrrolidone (PVP), polyethylene oxide (PEO) hydroxyethyl cellulose (HEC), polyethylene glycol (PEG), pullulan, sodium alginate, xanthan gum, tragancanth gum, guar gum, acacia gum, arabic gum, polyacrylic acid, maltodextrin, methylmethacrylate copolymer, carboxyvinyl copolymers, starch and gelatin.

The pouch is typically completely insoluble in saliva. Suitable insoluble pouch materials include, but are not limited to, fiber, paper, water-insoluble polymers, cloth and fabric. Suitable soluble pouch materials include, but are not limited to, water-soluble polymers such as polyethylene oxide (PEO), hydroxypropyl cellulose (HPC) and hydroxypropyl methylcellulose (HPMC).

The pouch may be formed by, for example, folding a single sheet on itself or bringing two or more sheets together and sealing the edges. The edges may initially be partially sealed to provide an open pouch in which the pouch content may be placed before completely sealing the pouch closed. The sheets may be the same thickness or different thicknesses.

The pouch is porous. In some examples, at least 50% of the pores have a diameter of from 50 µm to 200 µm, such as from 100 µm to 175 µm or from 125 µm or 150 µm. In some examples, at least 50% of the pores have a diameter of at least 100 µm. For example, in some examples at least 55%, 60%, 65%, 70%, 75%, 80%, 85%, 90%, 95% or 100% of the pores have such diameters.

The pouch may be coloured or include markings, such as brand logos and text, to improve user perception. The pouch may be partially or completely coloured by a colourant.

In some examples, a flavourant may be applied to the pouch. This may be instead of or in addition to any flavourant contained by the content of the pouch. In examples where the pouch comprises a nicotinic compound spray coating, a flavourant may also be applied to the pouch by spraying.

The pouch content (i.e. the ingredients, material and/or substances enclosed within the pouch) may occupy substantially all of the internal volume of the pouch. The content may occupy 80%, 85%, 90%, 95% or 100% of the internal volume of the pouch. The contents may comprise a solid material to provide physical integrity, such as an organic material (e.g. plant material) or an inorganic material. Such solid materials may naturally or inherently contain one or more biologically/pharmacologically active compounds and/or additives.

In a **second aspect,** the present disclosure provides a method of manufacturing a smokeless article for oral delivery. In some examples, the smokeless article is a smokeless article according to the first aspect. The alternatives and optional features described with reference to the first aspect are therefore equally applicable to the second aspect.

In some examples, the method comprises: i) providing a pouch content; ii) enclosing the content in a pouch; and iii) sealing the pouch.

In some examples, providing the pouch content (step i)) comprises mixing dulcitol with a nicotinic compound (e.g. nicotine), filler material and water. In some examples, providing the pouch content (step i)) comprises: a) providing filler material; b) adding water to the filler material and mixing; c) adding dulcitol to the mixture obtained in step b) and mixing; and d) adding a nicotinic compound to the mixture obtained in step c) and mixing. In some examples, step c) may be carried out before step b). That is, in some examples, dulcitol is added to the filler material before the water is added. In some examples, the pouch content resulting from step i) is isotropic. That is, the components of the content are evenly dispersed (e.g. they may appear to be homogeneously mixed on the macroscopic scale). In some examples, components are mixed using a blender or a food processor or similar. In some examples, providing a pouch content (step i)) further comprises adding at least one of a flavourant, humectant, sweetener, colourant, pH adjuster and solvent. In some examples, the at least one of a flavourant, humectant, sweetener, colourant, pH adjuster and solvent are added to the mixture obtained in step d) and mixed.

In some examples, the components are mixed in a high-shear mixing apparatus. Suitable mixing apparatus is known to the skilled person.

Sealing the pouch (step iii)) may comprise thermally or chemically sealing the pouch material to enclose the content.

In some examples, the method further comprises a step of applying a flavourant to the pouch. In some examples, the step of applying a flavourant to the pouch comprises spraying the pouch with a flavourant.

In some examples, the method further comprises a step of spraying the pouch with water.

In a **third aspect,** the present disclosure provides a smokeless article for oral delivery obtained or obtainable by the method of the second aspect.

Any alternatives and optional features described with reference to the first and second aspect are equally applicable to the third aspect.

In a **fourth aspect,** the present disclosure provides a kit comprising a plurality of smokeless articles according to the first aspect, or a plurality of smokeless articles produced according to the second aspect.

In some examples, the kit further comprises a container, wherein the container removably encloses said plurality of smokeless articles.

The preceding summary is provided for purposes of summarizing some examples to provide a basic understanding of aspects of the subject matter described herein. Accordingly, the above-described features should not be construed to narrow the scope of the subject matter described herein in any way. Moreover, the above and/or following examples may be combined in any suitable combination to provide further examples, except where such a combination is clearly impermissible or expressly avoided. Other features, aspects, and advantages of the subject matter described herein will become apparent from the following text and the accompanying figures.

### BRIEF DESCRIPTION OF THE FIGURES

Aspects, features and advantages of the present disclosure will become apparent from the following description of examples in reference to the appended figures in which like numerals denote like elements.
**Figure 1** shows a cross sectional view of a first embodiment of a smokeless article.
**Figure 2** shows a cross sectional view of a second embodiment of a smokeless article.
**Figure 3** shows a cross sectional view of a third embodiment of a smokeless article.

### DETAILED DESCRIPTION OF EMBODIMENTS

It is to be understood that the present disclosure, which includes the specification and claim(s), is not limited by specific construction details or process steps. Rather, it will be clear to those skilled in the art that the systems, apparatuses, and methods described herein can be embodied and practiced in various alternative ways without departing from the scope of the invention.

Unless defined otherwise, scientific and technical terms used herein have their meanings commonly understood by those skilled in the art and that known techniques and procedures may be performed according to conventional methods.

In the present disclosure, the terms "a" and "an" may mean "one", "one or more", "at least one", and "one or more than one" unless the context clearly indicates otherwise. Likewise, plural terms shall include the singular unless otherwise required by context.

In the present disclosure, the term "or" means an inclusive "and/or" unless explicitly indicated to refer to alternatives only or unless the alternatives are mutually exclusive.

In the present disclosure, the terms "comprising, "having," "including," or "containing" (and any forms thereof, such as "comprise" and "comprises," "have" and "has," "includes" and "include," or "contains" and "contain," respectively) are inclusive or open-ended and do not exclude additional, unrecited elements or method steps.

Unless stated otherwise, the features of examples disclosed herein, and of the claims, may be integrated together in any suitable arrangement such that combinations of features are not limited by the described forms, particularly the form (e.g. numbering) of example(s), embodiment(s), or dependency of claim(s). This also applies to the phrase "in one example", "according to an example" and the like, which are merely a stylistic form of wording not to be construed as limiting the features to a separate embodiment. This is to say, a reference to 'an,' 'one,' or 'some' examples(s) may be a reference to any one or more, and/or all examples, or combination(s) thereof, disclosed. Also, similarly, reference to "the" example may not be limited to the immediately preceding embodiment. Further, all references to one or more embodiments or examples are to be construed as non-limiting to the claims.

The present disclosure may be better understood in view of the following explanations, wherein the terms used that are separated by "or" may be used interchangeably.

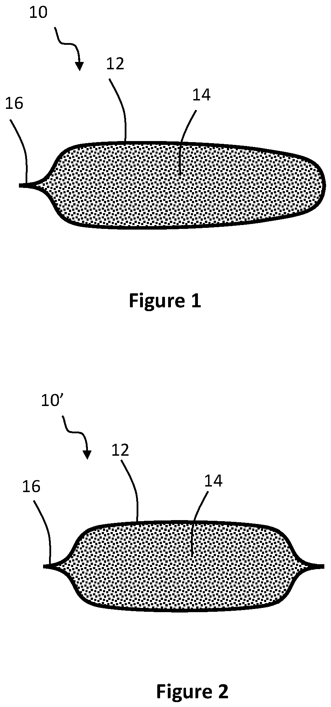

As shown in Figure 1 there is provided a first embodiment of a smokeless article 10 having a pouch 12 containing a pouch content 14. The pouch content 14 comprises dulcitol, a nicotinic compound such as nicotine, filler material and water. The pouch 12 is substantially rectangular. The pouch 12 is formed from a single sheet of material and is substantially filled by the pouch content 14. The pouch 12 has a seal 16 along each of the three edges where the inner face of the single sheet meets itself to seal the pouch content 14 in the pouch 12.

Figure 2 shows a second embodiment of a smokeless article 10' having a pouch 12 containing a pouch content 14. The pouch content 14 comprises dulcitol, a nicotinic compound such as nicotine, filler material and water. The pouch 12 is substantially circular. The pouch 12 is formed from two opposing sheets of material and is substantially filled by the pouch content 14. The pouch has a circumferential seal 16 along the edges where the two opposing sheets of material meet to seal the pouch content 14 in the pouch 12.

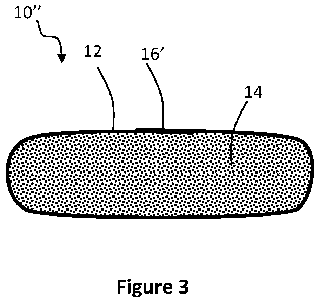

Figure 3 shows a third embodiment of a smokeless article 10" that, like the first embodiment, has a pouch 12 made from a single sheet of material. However, one of the three seals 16' is formed by an overlap of the inner face and the outer face of the single sheet meet to seal the pouch content 14 in the pouch 12. The remaining two seals at opposing ends of the pouch 12 are formed where the inner face of the single sheet meets itself.

### Example formulations

The example formulations contained the following components.

| **Material** | **Approx %w/w values** | |
|---|---|---|
| | **lower range** | **upper range** |
| Nicotine | 0.5 | 2.4 |
| Water | 35 | 40 |
| Filler material 1 | 20 | 32 |
| Dulcitol | 1 | 30 |
| Filler material 2 | 0 | 15 |
| Humectant 1 | 4 | 10 |
| Humectant 2 | 0 | 3 |
| pH stabiliser/adjuster | 0.1 | 0.6 |
| Flavour mix | 2 | 5 |

## Claims

1. A smokeless article for oral delivery, the article comprising a pouch,
wherein the pouch encloses a pouch content, the content comprising:
dulcitol in an amount of about 35% by weight of the total content or less;
a nicotinic compound in an amount from about 0.1 to about 5% by weight of the total content;
at least one filler material in an amount from about 1 to about 80% by weight of the total content; and
water in an amount of about 1 to about 65% by weight of the total content.

2. The smokeless article according to claim 1, wherein the content further comprises a sugar alcohol selected from the group xylitol, sorbitol, mannitol, erythritol, isomalt, glycerine, maltitol, lactitol and inositol.

3. The smokeless article according to claim 2, wherein the sugar alcohol is xylitol.

4. The smokeless article according to any of claims 1 to 3, wherein the content comprises dulcitol in an amount of 15 to 30% by weight of the total content.

5. The smokeless article according to any of claims 1 to 4, wherein the nicotinic compound is selected from nicotine, nicotine salts, nicotine complexes and nicotine solvates, optionally wherein the nicotinic compound is nicotine.

6. The smokeless article according to any of claims 1 to 5, wherein the water is present in an amount of greater than 5% by weight of the total content.

7. The smokeless article according to any of claims 1 to 6, wherein the content further comprises tobacco in an amount of 0.1% by weight of the total content.

8. The smokeless article according to any of claims 1 to 6, wherein the content is substantially free from tobacco.

9. The smokeless article according to any of claims 1 to 8, wherein the filler material comprises fibrous material,

10. The smokeless article according to claim 9, wherein the filler material comprises one or more selected from the group consisting of a plant fibre, a bio-derived fibre or a synthetic fibre.

11. The smokeless article according to any of claims 1 to 10, wherein the content further comprises at least one of a flavourant, humectant, sweetener, colourant, pH adjuster and solvent.

12. The smokeless article according to any of claims 1 to 11, wherein the content has a bulk density of about 0.8 g/cm³ or less.

13. A method of preparation of a smokeless article according to any one of claims 1 to 12, the method comprising the steps:
i) providing a pouch content;
ii) enclosing the pouch content in a pouch; and
iii) sealing the pouch.

14. The method according to claim 13, wherein providing a pouch content comprises the steps:
a) providing the filler material;
b) adding water to the filler material and mixing;
c) adding dulcitol to the mixture obtained in step b) and mixing;
d) adding a nicotinic compound to the mixture obtained in step c) and mixing.

15. The method of claim 14, wherein providing a pouch content further comprises a step of adding at least one of a flavourant, humectant, sweetener, colourant, pH adjuster and solvent, optionally this step comprises adding to the mixture obtained in step d) and mixing.
